(19) Europäisches Patentamt
European Patent Office
Office européen des brevets

(11) **EP 2 984 095 B1**

(12) **FASCICULE DE BREVET EUROPEEN**

(45) Date de publication et mention de la délivrance du brevet:
**01.11.2017 Bulletin 2017/44**

(51) Int Cl.:
***C07G 1/00*** *(2011.01)*    ***C08H 7/00*** *(2011.01)*
***C08H 8/00*** *(2010.01)*

(21) Numéro de dépôt: **14720666.8**

(22) Date de dépôt: **07.04.2014**

(86) Numéro de dépôt international:
**PCT/FR2014/050831**

(87) Numéro de publication internationale:
**WO 2014/167233 (16.10.2014 Gazette 2014/42)**

(54) **PROCEDE DE TRANSFORMATION DE BIOMASSES LIGNOCELLULOSIQUES EN MOLECULES MONO OU POLY-OXYGENEES**

VERFAHREN ZUR UMWANDLUNG VON LIGNOCELLULOSEBIOMASSEN IN MONO- ODER POLYOXYGENIERTE MOLEKÜLE

METHOD FOR CONVERTING LIGNOCELLULOSIC BIOMASSES INTO MONO- OR POLY-OXYGENATED MOLECULES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.04.2013 FR 1353198**

(43) Date de publication de la demande:
**17.02.2016 Bulletin 2016/07**

(73) Titulaire: **IFP Energies nouvelles**
**92500 Rueil-Malmaison (FR)**

(72) Inventeurs:
• **DELCROIX, Damien**
**38550 St Maurice L Exil (FR)**
• **VALLEE, Christophe**
**38360 Sassenage (FR)**
• **CABIAC, Amandine**
**69700 Givors (FR)**
• **GUILLON, Emmanuelle**
**69390 Vourles (FR)**

(56) Documents cités:
**US-A1- 2011 313 208**

• **ZHIJUN TAI ET AL: "Temperature-controlled phase-transfer catalysis for ethylene glycol production from cellulose", CHEMICAL COMMUNICATIONS, vol. 48, no. 56, 1 janvier 2012 (2012-01-01), page 7052, XP055070686, ISSN: 1359-7345, DOI: 10.1039/c2cc32305b**

**Description**

**DOMAINE DE L'INVENTION**

L'invention concerne un procédé de transformation de biomasse lignocellulosique ou de cellulose mettant en oeuvre de manière simultanée une combinaison originale d'un ou plusieurs catalyseurs homogènes et d'un ou plusieurs catalyseurs hétérogènes. L'utilisation de ces catalyseurs permet d'obtenir directement des produits valorisables mono- ou polyoxygénés et de limiter la formation de produits non valorisables. Par produits non valorisables, on entend les humines solubles et non solubles, c'est à dire des produits de poids moléculaire important issus de réactions de condensation non désirées de sucres et de leurs dérivés.

**ART ANTERIEUR**

Depuis quelques années, il existe un vif regain d'intérêt pour l'incorporation de produits d'origine renouvelable au sein des filières carburant et chimie, en complément ou en substitution des produits d'origine fossile. Une voie possible est la conversion de la cellulose contenue dans la biomasse lignocellulosique en produits ou intermédiaires chimiques, comme des produits contenant de une à six fonctions hydroxyles, le n-propanol, l'éthylène glycol, le propylène glycol, le glycérol, le 1,2-butanediol ou le 1,2-hexanediol.

Le terme biomasse lignocellulosique (BLC) ou lignocellulose englobe plusieurs produits présents en quantités variables selon son origine : la cellulose, l'hémicellulose et la lignine. L'hémicellulose et la cellulose constituent la partie carbohydrate de la lignocellulose. Ce sont des polymères de sucres (pentoses et hexoses). La lignine est une macromolécule riche en motifs phénoliques. Par biomasse lignocellulosique, on entend par exemple les produits issus de l'exploitation forestière et les sous-produits issus de l'agriculture comme la paille ainsi que certains végétaux dédiés à haut rendement agricole comme le miscanthus ou le peuplier.

La production de produits chimiques à partir de biomasse lignocellulosique permet à la fois de réduire la dépendance énergétique vis-à-vis du pétrole et de préserver l'environnement à travers la diminution des émissions de gaz à effet de serre sans utiliser de ressources destinées aux usages alimentaires.

La transformation directe de biomasse lignocellulosique ou de cellulose en produits ou intermédiaires chimiques, notamment mono- ou polyoxygénés est une voie particulièrement intéressante. Par transformation directe, on entend la transformation en une étape de la biomasse lignocellulosique ou de la cellulose, éventuellement prétraitée, vers des produits valorisables mono- ou polyoxygénés.

La valorisation de la biomasse lignocellulosique ou de la cellulose contenue dans la biomasse par l'utilisation d'une combinaison de catalyseur homogène et hétérogène est largement décrite dans la littérature.

La demande de brevet US2009/0326286 décrit l'hydrolyse et l'hydrogénation de biomasses lignocellulosiques en monosaccharides en présence d'un catalyseur homogène et d'un catalyseur hétérogène. Le catalyseur homogène est décrit comme un acide minéral ou organique de Brønsted choisi de préférence parmi les acides $H_2SO_4$, $H_3PO_4$, $H_3PO_3$, $H_3PO_2$ et $CH_3COOH$. Le catalyseur hétérogène est à base de charbon activé, silice, zéolites ou des polymères de haut poids moléculaires, sur lequel est déposé un métal de transition choisi parmi le ruthénium, le nickel, le platine et le palladium à des teneurs comprises entre 0,1% et 5,5% poids par rapport à la masse totale de catalyseur hétérogène. Les produits formés et les rendements associés ne sont pas précisés.

Sels et al. (Chem. Comm. 2010, 46, 3577-3579) étudient la transformation de cellulose en hexitols (sorbitol+mannitol) en présence d'un catalyseur homogène et d'un catalyseur hétérogène. Les catalyseurs acides homogènes utilisés sont $H_2SO_4$ et $H_4SiW_{12}O_{40}$. Le catalyseur hétérogène est du Ru/C. La conversion de la cellulose microcristalline est respectivement de 50% et 80% avec ces deux acides pour une réaction dans l'eau à 190°C et sous 50 bars d'$H_2$ pendant 24h (pH(25°C)=2). Les rendements associés en hexitols sont de 48% et 13%. La cellulose broyée a une réactivité exacerbée avec une conversion totale en 1h dans les mêmes conditions opératoires et un rendement en hexitol de 87%.

Avec une optique un peu différente, Zhang et al. (Chem. Comm., 2012, 48, 7052-7054) combinent l'acide tungstique $H_2WO_4$, non soluble dans l'eau à température ambiante, et le catalyseur hétérogène Ru/C pour la conversion de la cellulose en éthylène glycol dans l'eau à 245°C sous 60 bars $H_2$. La particularité de ce système est que l'acide tungstique se solubilise à chaud et passe de l'hétérogène à l'homogène pendant la montée à 245°C. Le rendement en éthylène glycol atteint 59% avec conversion totale de la cellulose en 30 min.

Plus récemment, C. Liang (Green Chem., 2013, 15, 891-895) décrit une combinaison de catalyseurs pour la production d'éthylène glycol à partir de cellulose, dans l'eau à 245°C sous 60 bars $H_2$. L'ajout de l'hydroxyde de calcium $Ca(OH)_2$ en association avec le catalyseur hétérogène CuCr permet d'augmenter le rendement en éthylène glycol de la réaction de 5% à 30%. Le rendement en propylène glycol reste quant à lui stable autour de 30-35%.

Enfin, en 2009, R. Raines (JACS, 2009, 131, 1979-1985) a décrit la transformation de sucres, de cellulose et de lignocellulose en 2,5-diméthylfurane en deux étapes. La première étape est réalisée dans un milieu liquide ionique à base de DMA-LiCl/[EMIM]Cl à 140°C pendant 2h et est catalysée par un mélange de $CrCl_3$ et HCl tous deux à 10% molaire relativement à la cellulose. A l'issue de cette première étape, une étape de purification par chromatographie d'exclusion stérique est mise en oeuvre et permet d'éliminer les ions chlorures du milieu de manière à éviter l'empoisonnement du catalyseur utilisé dans la deuxième étape. La deuxième étape peut ainsi avoir lieu et implique la transformation de la solution purifiée en présence d'un catalyseur à base de cuivre déposé sur du Ru/C sous hydrogène, dans le 1-butanol à 220°C pendant 10h pour former du 2,5-diméthylfurane.

Excepté ces deux derniers exemples rapportant respectivement l'utilisation d'un sel de métal alcalino-terreux et l'utilisation, non simultanée, dans une première étape d'un catalyseur homogène comprenant un sel métallique et d'un deuxième catalyseur homogène à base d'acide de Brønsted puis dans une deuxième étape opérant dans des conditions différentes de la première, d'un catalyseur hétérogène, il n'est pas décrit dans la littérature de procédé qui permette une transformation directe de la cellulose ou plus largement de la biomasse lignocellulosique, éventuellement prétraitée, en produits valorisables mono ou polyoxygénés, par mise en contact de la biomasse lignocellulosique, simultanément, au sein d'un même milieu réactionnel, avec une combinaison d'un ou plusieurs catalyseurs homogènes et d'un ou plusieurs catalyseurs hétérogènes du type de ceux décrits dans la présente invention.

La demande de brevet WO2012/035160 de BIOeCON International Holding décrit par ailleurs l'hydrolyse et l'hydrogénation du cellobiose et de la cellulose dans un milieu liquide ionique comme solvant à des températures comprise entre 80 et 220°C en présence d'un catalyseur hétérogène, et de préférence en présence du Ru/C. Le solvant liquide ionique est de manière préférée un sel inorganique hydraté et en particulier le $ZnCl_2.4H_2O$ avec un rapport massique cellobiose/$ZnCl_2.4H_2O$ de 1/12 et un rapport massique cellulose/$ZnCl_2.4H_2O$ de 1/24. La conversion maximale de la cellulose ainsi obtenue après 6h est de 60%. Le sorbitol est formé avec un rendement de 55%. La transformation est accompagnée de la formation d'humines. Les mêmes travaux ont été reportés très récemment en se focalisant sur le cellobiose dans la publication suivante : Makkee et al. (Catal. Sci. Technol., doi 10.1039/c3cy20808g). Le document US2011/313208 décrit un procédé de production de polyol a partir d'une charge comprenant au moins un saccharide par mise en contact ladite charge avec de l'eau, de l'hydrogène et un système catalytique.

Les travaux du demandeur ont permis de mettre en évidence que, de manière surprenante, la mise en contact de la biomasse lignocellulosique ou de la cellulose, simultanément avec une combinaison d'un ou plusieurs catalyseurs homogènes et d'un ou plusieurs catalyseurs hétérogènes, dans une même enceinte réactionnelle opérant dans des conditions opératoires spécifiques permettait d'obtenir directement des produits valorisables mono-ou polyoxygénés et de diminuer la teneur en produits non valorisables, tels que les humines.

## RESUME DE L'INVENTION

Un objet de la présente invention est donc de fournir un procédé de transformation de biomasse lignocellulosique ou de cellulose en composés mono ou polyoxygénés, dans lequel la biomasse lignocellulosique ou la cellulose est mise en contact, simultanément, avec une combinaison d'un ou plusieurs catalyseurs homogènes et d'un ou plusieurs catalyseurs hétérogènes, dans une même enceinte réactionnelle, en présence d'au moins un solvant, ledit solvant étant de l'eau seule ou en mélange avec au moins un autre solvant, sous atmosphère réductrice, et à une température comprise entre 80°C et 250°C, et à une pression comprise entre 0,5 MPa et 20 MPa,

- ledit ou lesdits catalyseur(s) homogène(s) comprenant un sel métallique hydraté ou non de formule générale $MX_n.n'H_2O$ dans lequel M est un métal choisi parmi les métaux des groupes 3 à 16 de la classification périodique, n est un nombre entier compris entre 1 et 6 et n' est un nombre entier compris entre 0 et 6 et X est au moins un anion choisi parmi les halogénures, les nitrates, les carboxylates, les halogénocarboxylates, les acétylacétonates, les alcoolates, les phénolates, substitués ou non, les sulfates, les alkylsulfates, les phosphates, les alkylphosphates, les halogénosulfonates, les alkylsulfonates, les perhalogénoalkylsulfonates, les bis(perhalogénoalkylsulfonyl)amidures, les arènesulfonates, substitués ou non par des groupements halogènes ou halogénoalkyles, lesdits anions X pouvant être identiques ou différents dans le cas où n est supérieur à 1,

- ledit ou lesdits catalyseur(s) hétérogène(s) comprenant au moins un métal choisi parmi les métaux des groupes 6 à 11 et les métaux du groupe 14 de la classification périodique et un support choisi parmi les oxydes des éléments choisis parmi l'aluminium, le titane, le silicium, le zirconium, le cérium, et le niobium, et les oxydes mixtes choisis parmi les aluminates de zinc, de cuivre et de cobalt, lesdits oxydes pouvant être dopés ou non par au moins un composé métallique choisi parmi le tungstène, l'étain, le molybdène et l'antimoine, pris seuls ou en mélange, les aluminosilicates cristallisés ou non, les aluminophosphates et les composés carbonés amorphes ou cristallisés.

Dans la présente invention, il est fait référence à la nouvelle notation de la classification périodique des éléments : Handbook of Chemistry and Physics, 76ième édition, 1995-1996.

Dans la présente invention, on entend par catalyseur homogène, un catalyseur soluble dans le milieu réactionnel. On entend par catalyseur hétérogène, un catalyseur non soluble dans le milieu réactionnel.

Un avantage de la présente invention est de permettre l'obtention directe de produits valorisables mono-ou polyoxygénés tout en limitant la formation de produits non valorisables tels que les humines solubles et non solubles, c'est à dire des produits de poids moléculaire important issus de condensations non désirées de sucres et de leurs dérivés.

Un autre avantage de la présente invention est de permettre à la fois, l'amélioration de la conversion et l'amélioration de la cinétique de conversion de la biomasse lignocellulosique ou de la cellulose par l'utilisation simultanée dans une même enceinte réactionnelle opérant sous atmosphère réductrice, de la combinaison de catalyseurs homogène et hétérogène tels que revendiqués.

## DESCRIPTION DETAILLEE DE L'INVENTION

### La charge

La charge traitée dans le procédé selon l'invention est la biomasse lignocellulosique ou la cellulose.

La biomasse lignocellulosique est essentiellement constituée de trois constituants naturels présents en quantités variables selon son origine : la cellulose, l'hémicellulose et la lignine.

La cellulose $(C_6H_{10}O_5)_n$ représente la majeure partie (40-60%) de la composition de la biomasse lignocellulosique. La cellulose est un homopolymère linéaire composé de nombreuses unités de D-Anhydroglucopyranose (AGU) reliées entre elles par des liaisons glycosidiques $\beta$-$(1{\rightarrow}4)$. Le motif de répétition est le dimère cellobiose.

La cellulose est insoluble dans l'eau à température et pression ambiantes.

L'hémicellulose est le deuxième carbohydrate en quantité après la cellulose et constitue 20 à 40% en poids de la biomasse lignocellulosique. Contrairement à la cellulose, ce polymère est constitué en majorité de monomères de pentoses (cycles à 5 atomes) et hexoses (cycles à 6 atomes). L'hémicellulose est un hétéropolymère amorphe avec un degré de polymérisation inférieur à celui de la cellulose (30-100).

La lignine est une macromolécule amorphe présente dans les composés lignocellulosiques dans des proportions variables selon l'origine du matériau (paille ~ 15%, bois : 20-26%). Sa fonction est le renforcement mécanique, l'hydrophobisation et le soutien des végétaux. Cette macromolécule riche en motifs phénoliques peut être décrite comme résultant de la combinaison de trois unités monomères de type propyl-méthoxy-phénols. Sa masse molaire varie de 5000 g/mol à 10000 g/mol pour les bois durs et atteint 20000 g/mol pour les bois tendres.

La matière première lignocellulosique peut avantageusement être constituée de bois ou de déchets végétaux. D'autres exemples non limitatifs de matière biomasse lignocellulosique sont les résidus d'exploitation agricole tels que par exemple la paille, les herbes, les tiges, les noyaux, ou les coquilles, les résidus d'exploitation forestière tels que les produits de première éclaircie, les écorces, les sciures, les copeaux, ou les chutes, les produits d'exploitation forestière, les cultures dédiées (taillis à courte rotation), les résidus de l'industrie agro-alimentaire tels que les résidus de l'industrie du coton, du bambou, du sisal, de la banane, du maïs, du panicum virgatum, de l'alfalfa, de la noix de coco, ou de la bagasse, les déchets organiques ménagers, les déchets des installations de transformation du bois et les bois usagés de construction, de la pâte à papier, du papier, recyclé ou non.

La charge utilisée dans le procédé selon l'invention est de la biomasse lignocellulosique ou de la cellulose. La cellulose utilisée peut être cristalline ou amorphe. La charge du procédé selon l'invention peut également avantageusement comprendre le cellobiose et des polymères amorphes de glucose, comme l'amidon ou du glucose. Enfin la charge peut être du saccharose.

La charge biomasse lignocellulosique peut avantageusement être utilisée sous sa forme brute, c'est-à-dire dans son intégralité de ces trois constituants cellulose, hémicellulose et lignine. La biomasse brute se présente généralement sous forme de résidus fibreux ou poudre. Elle peut également avantageusement être broyée ou déchiquetée pour permettre son transport.

La charge biomasse lignocellulosique peut avantageusement aussi être utilisée sous sa forme prétraitée, c'est-à-dire sous une forme contenant au moins une partie cellulosique après extraction de la lignine et/ou de l'hémicellulose.

La biomasse subit de préférence un prétraitement afin d'augmenter la réactivité et l'accessibilité de la cellulose au sein de la biomasse avant sa transformation. Ces prétraitements sont de nature mécanique, thermochimique, thermo-mécanico-chimique et/ou biochimique et provoquent la décristallisation de la cellulose, une diminution du degré de polymérisation de la cellulose, la solubilisation de l'hémicellulose et/ou de la lignine et/ou de la cellulose ou l'hydrolyse partielle de l'hémicellulose et/ou de la cellulose suivant le traitement.

La charge biomasse lignocellulosique peut également être prétraitée afin d'être sous la forme d'oligomères hydrosolubles. Ces prétraitements sont de nature mécanique, thermochimique, thermo-mécanico-chimique et/ou biochimique. Ils provoquent la décristallinisation et la solubilisation de tout ou partie de la cellulose sous forme d'oligomères hydrosolubles.

Les traitements mécaniques vont au delà du simple déchiquetage car ils modifient la structure chimique des constituants. Ils améliorent l'accessibilité et la réactivité de la cellulose par sa décristallinisation et par l'augmentation de la surface d'échange. Les traitements mécaniques incluent la réduction de la taille des fibres ou particules élémentaires, par exemple par mise en copeaux de la biomasse à l'aide d'une coupeuse, par broyage de la biomasse (ajustement de la granulométrie), déstructuration des copeaux sur presse ou défibrage par abrasion des copeaux, après préchauffage. Les traitements mécaniques peuvent être opérés en mode décentralisé près de la production de la biomasse ou en mode centralisé alimentant directement la transformation.

Les traitements thermochimiques incluent la cuisson de la biomasse à haute température (150-170°C) en milieu acide dilué (principalement acide sulfurique, mais aussi acide phosphorique, acide acétique ou acide formique), en milieu alcalin (soude, sulfites, chaux...), en milieu oxydant (oxydation humide à l'air ou à l'oxygène ; peroxyde en milieu alcalin ; acide peracétique) et en milieux non conventionnels comme les liquides ioniques (par exemple l'acétate de 1-éthyl-3-méthylimidazolium [emim][OAc]) ou les sels inorganiques hydratés ($FeCl_3.6H_2O$, $ZnCl_2.,5H_2O$) utilisés comme solvants. Les autres traitements thermochimiques incluent des traitements aux solvants (éthanol à chaud) ou la torréfaction qui peut se définir comme une pyrolyse à température modérée et temps de séjour contrôlé car elle s'accompagne d'une destruction partielle de la matière lignocellulosique. Les technologies connues pour la torréfaction sont par exemple le four tournant, le lit mobile, le lit fluidisé, la vis sans fin chauffée, le contact avec des billes métalliques apportant la chaleur. Ces technologies peuvent éventuellement utiliser un gaz circulant à co ou contre-courant comme de l'azote ou tout autre gaz inerte dans les conditions de la réaction.

Les traitements thermo-mécanico-chimiques incluent les traitements à la vapeur (explosion à la vapeur appelée encore hydrolyse flash ou "steam-explosion"), le traitement AFEX (ammonia fiber explosion) à l'ammoniaque ou l'extrusion bi-vis avec des réactifs chimiques divers.

Le prétraitement permet de préparer la biomasse lignocellulosique en séparant la partie carbohydrate de la lignine et ajustant la taille des particules de biomasse à traiter. La taille des particules de biomasse après le prétraitement est généralement inférieure à 5 mm, de préférence inférieure à 500 microns.

## Les catalyseurs

Conformément à l'invention, la biomasse lignocellulosique ou la cellulose est mise en contact dans le procédé selon l'invention, simultanément, avec une combinaison d'un ou plusieurs catalyseurs homogènes et d'un ou plusieurs catalyseurs hétérogènes, dans une même enceinte réactionnelle, en présence d'au moins un solvant, ledit solvant étant de l'eau seule ou en mélange avec au moins un autre solvant, sous atmosphère réductrice, et à une température comprise entre 80°C et 250°C, et à une pression comprise entre 0,5 MPa et 20 MPa.

Un critère essentiel de la présente invention réside dans la mise en contact de ladite charge dans les conditions opératoires telles que revendiquées, simultanément, avec une combinaison d'un ou plusieurs catalyseurs homogènes et d'un ou plusieurs catalyseurs hétérogènes, au sein d'une même enceinte réactionnelle.

En effet, les réactions mises en jeu dans le procédé de transformation de la biomasse lignocellulosique ou de la cellulose ne sont pas des réactions successives du fait de l'utilisation et du fonctionnement simultanés d'une combinaison d'un ou plusieurs catalyseurs homogènes et d'un ou plusieurs catalyseur hétérogène, dans une même enceinte réactionnelle. La solubilisation de la cellulose induite par le ou les catalyseurs homogènes et la transformation des produits ainsi dissouts par le ou les catalyseurs hétérogènes se fait donc de manière concomitante et complémentaire. Il est ainsi possible de tirer avantage de cette compatibilité entre les catalyseurs homogènes et hétérogènes pour s'affranchir de tout travail intermédiaire de traitement ou de purification, synonymes de coûts supplémentaires de procédé et de pertes de matière importants associés à cette étape.

De préférence, ledit procédé selon l'invention n'opère pas en deux étapes successives.

De préférence, la biomasse lignocellulosique ou la cellulose est mise en contact, simultanément, avec une combinaison d'un ou plusieurs catalyseurs homogènes et d'un catalyseur hétérogène, dans une même enceinte réactionnelle.

De manière plus préférée, la biomasse lignocellulosique ou la cellulose est mise en contact, simultanément, avec une combinaison d'un catalyseur homogène et d'un catalyseur hétérogène, dans une même enceinte réactionnelle.

Conformément à l'invention, ledit ou lesdits catalyseur(s) homogène(s) comprennent un sel métallique hydraté ou non de formule générale $MX_n.n'H_2O$ dans lequel M est un métal choisi parmi les métaux des groupes 3 à 16 de la classification périodique, n est un nombre entier compris entre 1 et 6 et n' est un nombre entier compris entre 0 et 6 et X est au moins un anion choisi parmi les halogénures, les nitrates, les carboxylates, les halogénocarboxylates, les acétylacétonates, les alcoolates, les phénolates, substitués ou non, les sulfates, les alkylsulfates, les phosphates, les alkylphosphates, les halogénosulfonates, les alkylsulfonates, les perhalogénoalkylsulfonates, les bis(perhalogénoalkylsulfonyl)amidures, les arènesulfonates, substitués ou non par des groupements halogènes ou halogénoalkyles, lesdits anions X pouvant être identiques ou différents dans le cas où n est supérieur à 1.

Ledit métal M choisi dans les groupes 3 à 16 de la classification périodique du catalyseur homogène selon invention est de préférence choisi parmi les métaux suivants : Al, Sc, Ti, V, Cr, Mn, Fe, Co, Ni, Cu, Zn, Ga, Ge, Y, Zr, Nb, Mo, Tc, Ru, Rh, Pd, Ag, Cd, In, Sn, Sb, La, Hf, Ta, W, Re, Os, Ir, Pt, Au, Hg, Tl, Pb, Bi, Po, Ac, Rf, Db, Sg, Bh, Hs, Mt, Ds, Rg, Uub.

De manière préférée, ledit métal M est choisi parmi les métaux suivants : Al, Sc, Ti, Cr, Mn, Fe, Co, Ni, Cu, Zn, Al, Ga, Y, Zr, Mo, In, Sn, La, Hf, Ta, W, Pb, Bi.

De manière très préférée, ledit métal M est choisi parmi les métaux suivants : Cr, Mn, Fe, Co, Ni, Cu, Zn, Al, Ga, In, Cu, Zr, Bi, La et Sn. De manière plus préférée, ledit métal M est choisi parmi les métaux : Cr, Mn, Fe, Zn, Al, Sn, Cu, Zr, Bi et La.

De manière encore plus préférée, ledit métal M est choisi parmi les métaux : Fe, Zn, Al, Sn, Cu, et La.

Conformément à l'invention, dans la composition du sel métallique, le métal M choisi parmi les métaux cités est associé avec un ou plusieurs anions X, qui peuvent être identiques ou différents.

De préférence, l'anion X est au moins un anion choisi parmi les halogénures, les alkylsulfonates, les perhalogénoalkylsulfonate, les bis(perhalogénoalkylsulfonyl)amidures.

De préférence, l'halogénure est le fluorure, le chlorure, le bromure et le iodure.

De préférence, l'alkylsulfonate est le mésylate et le tosylate.

De préférence, le perhalogénoalkylsulfonate est le triflate.

De préférence, le bis(perhalogénoalkylsulfonyl)amidure est le bis(triflimide).

De manière très préférée, dans le cas où n est égal à 3, l'anion X est un chlorure.

Ledit ou lesdits catalyseur(s) homogène(s) préférés comprennent un sel métallique hydraté ou non de formule générale $MX_n.n'H_2O$, n et n' ayant les significations pré-citées, dans lequel M est un métal choisi parmi les métaux Cr, Mn, Fe, Co, Ni, Cu, Zn, Al, Ga, In, et Sn et X est au moins un anion choisi parmi les halogénures, les alkylsulfonates, les perhalogénoalkylsulfonate, les bis(perhalogénoalkylsulfonyl)amidures, lesdits anions X pouvant être identiques ou différents dans le cas où n est supérieur à 1.

Ledit ou lesdits catalyseur(s) homogène(s) encore plus préférés comprennent un sel métallique hydraté ou non de formule générale $MX_n.n'H_2O$, n et n' ayant les significations pré-citées, dans lequel M est un métal choisi parmi les métaux Cr, Fe, Zn, Al et Sn, et X est au moins un anion choisi parmi le fluorure, le chlorure, le bromure, le iodure, le mésylate, le tosylate, le triflate et le bis(triflimide), lesdits anions X pouvant être identiques ou différents dans le cas où n est supérieur à 1.

Dans le cas où plusieurs catalyseurs homogènes sont utilisés, lesdits catalyseur(s) homogène(s) sont avantageusement choisis parmi les sels métalliques hydratés ou non de formule générale $MX_n.n'H_2O$, n et n' ayant les significations pré-citées, dans lesquel M est un métal choisi parmi les métaux Cr, Fe, Zn, Al et Sn, et X est au moins un anion choisi parmi le fluorure, le chlorure, le bromure, le iodure, le mésylate, le tosylate, le triflate et le bis(triflimide), lesdits anions X pouvant être identiques ou différents dans le cas où n est supérieur à 1 et lesdits catalyseurs homogènes pouvant être identiques ou différents.

Conformément à l'invention, ledit ou lesdits catalyseurs hétérogènes comprennent au moins un métal choisi parmi les métaux des groupes 6 à 11 et les métaux du groupe 14 de la classification périodique et un support choisi parmi les oxydes des éléments choisis parmi l'aluminium, le titane, le silicium, le zirconium, le cérium, et le niobium, et les oxydes mixtes choisis parmi les aluminates de zinc, de cuivre et de cobalt, lesdits oxydes pouvant être dopés ou non par au moins un composé métallique choisi parmi le tungstène, l'étain, le molybdène et l'antimoine, pris seuls ou en mélange, les aluminosilicates cristallisés ou non, les aluminophosphates et les composés carbonés amorphes ou cristallisés.

Ledit métal choisi parmi les métaux des groupes 6 à 11 et les métaux du groupe 14 de la classification périodique du ou des catalyseurs hétérogènes selon invention sont de préférence choisis parmi les métaux suivants : Cr, Mo, W, Mn, Tc, Re, Fe, Ru, Os, Co, Rh, Ir, Ni, Pd, Pt, Cu, Ag, Au, Zn, Cd, et Hg, d'une part, et parmi : Ge, Sn et Pb d'autres part, pris seuls ou en mélange.

De manière préférée, ledit métal est choisi parmi les métaux Mo, W, Re, Ru, Co, Rh, Ir, Ni, Pd, Pt, Cu, d'une part et Sn, d'autre part, pris seuls et en mélange.

Selon un mode de réalisation préféré, les mélanges de métaux suivants sont préférés : NiSn, RePt, FePt, SnPt, CuPt, IrPt, CoPt, RhPt, OsPt, RuRe, PdRe, RuSn et RuPt. Selon un mode de réalisation très préférée, ledit métal est le platine.

De préférence, la teneur en métal sur ledit ou lesdits catalyseurs hétérogènes est avantageusement compris entre 0,01% et 10% poids et de manière préférée entre 0,1 et 5% poids par rapport à la masse totale dudit ou desdits catalyseurs hétérogènes.

Le ou les métaux du ou des catalyseurs hétérogènes selon invention sont avantageusement déposés sur un support.

Conformément à l'invention, ledit ou lesdits catalyseurs hétérogènes comprennent un support choisi parmi les oxydes des éléments choisis parmi l'alumine, le titane, la silice, la zircone, le cérium, le niobium, et les oxydes mixtes choisis parmi les aluminates de zinc, de cuivre et de cobalt, lesdits oxydes pouvant être dopés ou non par au moins un composé métallique choisi parmi le tungstène, l'étain, le molybdène et l'antimoine, pris seuls ou en mélange, les aluminosilicates cristallisés ou non, les aluminophosphates et les composés carbonés amorphes ou cristallisés.

De préférence, les aluminosilicates cristallisés ou non sont choisis parmi les zéolithes et les solides mésostructurés.

De préférence, les composés carbonés amorphes ou cristallisés sont choisis parmi les charbons actifs, les noirs de carbone, les nanotubes de carbone, les carbones mésostructurés et les fibres de carbone.

Dans le cas où ledit support est choisi parmi les oxydes des éléments choisis parmi l'aluminium, le titane, le silicium, le zirconium, le cérium, le niobium, dopés, un élément métallique de préférence choisi parmi le tungstène, l'étain, l'antimoine et le molybdène seul ou en mélange est avantageusement ajouté audit support. De préférence, la teneur en élément métallique choisi parmi le tungstène, l'étain, l'antimoine et le molybdène seul ou en mélange est avantageusement comprise entre 0,1% et 30% poids et de manière préférée entre 1 et 20% poids par rapport à la masse totale dudit catalyseur.

Ledit support est de préférence hydrothermalement stable, c'est à dire stable dans des conditions alliant l'eau et la température. Ainsi le support peut subir une étape de traitement visant à améliorer sa stabilité dans les conditions hydrothermales de la réaction. On peut citer par exemple la passivation de surface, le dépôt de film carboné, le dépôt d'oxyde.

Le dépôt du ou des métaux choisis parmi les groupes 6 à 11 et les métaux du groupe 14 de la classification périodique sur ledit support du ou des catalyseurs hétérogènes selon invention fait généralement intervenir un précurseur du ou des métaux. Par exemple il peut s'agir de complexes organiques métalliques, de sels de métaux comme les chlorures métalliques, les nitrates métalliques.

L'introduction du ou des métaux peut avantageusement être réalisée par toute technique connue de l'Homme du métier comme par exemple l'échange ionique, l'imprégnation à sec, l'imprégnation par excès, le dépôt en phase vapeur, etc. L'introduction de métal peut être réalisée avant ou après la mise en forme du support

L'étape d'introduction du ou des métaux peut avantageusement être suivie d'une étape de traitement thermique. Le traitement thermique est avantageusement réalisé entre 300°C et 700°C, sous atmosphère oxygène ou air. L'étape de traitement thermique peut être suivie d'un traitement de réduction en température. Le traitement thermique réducteur est avantageusement réalisé à une température comprise entre 200°C et 600°C sous flux ou atmosphère d'hydrogène. De préférence, ledit ou lesdits catalyseurs hétérogènes subissent également une étape de réduction in-situ c'est-à-dire dans le réacteur où se déroule la réaction, avant l'introduction de la charge réactionnelle. Ladite étape de réduction peut également avantageusement être réalisée ex-situ.

La taille des particules métalliques du ou des catalyseurs hétérogènes mis oeuvre dans le procédé selon l'invention est de manière préférée inférieure à 10 nm.

Le ou les catalyseurs hétérogènes utilisés dans la présente invention peuvent être sous forme de poudre, d'extrudés, de billes ou de pastilles. La mise en forme peut être réalisée avant ou après l'introduction du métal.

Le ou les catalyseurs hétérogènes utilisés dans la présente invention sont caractérisés par les techniques connues de l'homme du métier. On citera par exemple, pour caractériser la phase métallique, la microscopie à transmission.

## Procédé de transformation

Conformément à l'invention, le procédé de transformation de la biomasse lignocellulosique ou de la cellulose est mis en oeuvre dans une enceinte réactionnelle en présence d'au moins un solvant, ledit solvant étant de l'eau seule ou en mélange avec au moins un autre solvant, sous atmosphère réductrice, et à une température comprise entre 80°C et 250°C, et à une pression comprise entre 0,5 MPa et 20 MPa.

Le procédé est donc mis en oeuvre dans une enceinte réactionnelle comprenant au moins un solvant et dans laquelle la charge est mise en présence du système catalytique selon l'invention.

Conformément à l'invention, le procédé selon l'invention opère en présence d'au moins un solvant, ledit solvant étant de l'eau seule ou en mélange avec au moins un autre solvant.

Selon un mode de réalisation préféré, le procédé selon l'invention opère en présence d'eau en mélange avec au moins un alcool ou au moins un solvant organique, dans des conditions sub ou supercritique.

Les alcools sont avantageusement choisis parmi le méthanol, l'éthanol et les propanols.

Les solvants organiques peuvent avantageusement être choisis parmi le tétrahydrofurane et l'acétate d'éthyle.

Dans le cas où ledit procédé selon l'invention opère en présence d'eau en mélange avec au moins un autre solvant, le mélange de solvants comprend une teneur massique en eau supérieure à 5% poids et de manière préférée supérieure à 30% et de manière très préférée supérieure à 50% par rapport à la masse totale dudit mélange.

Selon un autre mode de réalisation, le procédé selon l'invention opère uniquement en présence d'eau.

De préférence, le procédé selon l'invention opère en présence d'au moins un solvant à l'exception des solvants choisis parmi les liquides ioniques.

Conformément à l'invention, le procédé de transformation de la biomasse lignocellulosique ou de la cellulose selon l'invention est réalisée sous atmosphère réductrice, de préférence sous atmosphère d'hydrogène. L'hydrogène peut être utilisé pur ou en mélange.

De préférence, ledit procédé selon l'invention opère à une température comprise entre 150°C et 240 °C, et à une pression comprise entre 2MPa et 10 MPa.

Généralement le procédé peut être opéré selon différents modes de réalisation. Ainsi, le procédé peut avantageusement être mise en oeuvre en discontinu ou en continu, par exemple en lit fixe. On peut opérer dans une enceinte réactionnelle fermée ou en réacteur semi-ouvert.

Le ou les catalyseur(s) homogène(s) sont avantageusement introduits dans l'enceinte réactionnelle à raison d'une quantité correspondant à un rapport massique biomasse/catalyseur(s) homogène(s) compris entre 1 et 1000 et de préférence entre 10 et 500.

Le ou les catalyseur(s) hétérogène(s) sont introduits dans l'enceinte réactionnelle à raison d'une quantité correspondant à un rapport massique biomasse/catalyseur(s) hétérogène(s) compris entre 1 et 1000, de préférence entre 1 et 500, de préférence entre 1 et 100, de préférence entre 1 et 50 et encore préférentiellement entre 1 et 25.

Le ou les catalyseur(s) hétérogène(s) introduit(s) dans le réacteur peu(ven)t subir une étape de traitement thermique réducteur avant l'introduction de la charge réactionnelle. Le traitement thermique réducteur est de préférence réalisé à une température comprise entre 150°C et 600°C sous flux ou atmosphère d'hydrogène.

La biomasse est introduite dans le procédé à raison d'une quantité correspondant à un rapport massique solvant/biomasse compris entre 1 et 1000, de préférence entre 1 et 500 et encore préférentiellement entre 5 et 100.

Si l'on choisit un procédé en continu, la vitesse massique horaire (débit de charge massique/masse de catalyseur(s) hétérogène(s)) est entre 0,01 et 5 $h^{-1}$, de préférence entre 0,02 et 2 $h^{-1}$.

## Les produits obtenus et leur mode d'analyse

Les produits de la réaction du procédé de transformation selon l'invention sont des composés mono ou polyoxygénés. Lesdits composés mono ou polyoxygénés sont solubles dans l'eau.

Lesdits composés mono ou polyoxygénés sont avantageusement constitués de monosaccharides et de leurs dérivés, d'oligosaccharides, et également de polymères solubles avantageusement formés par combinaisons successives des dérivés des monosaccharides.

Par monosaccharides, on désigne un carbohydrate ayant pour composition $C_nH_{2n}O_n$ où n est supérieur à 2, obtenu par hydrolyse totale de la cellulose, ou de l'hémicellulose, ou de l'amidon. Les monosaccharides sont des sucres simples qui sont produits par dépolymérisation complète de la cellulose et/ou hémicellulose, tels que en particulier, le glucose, le mannose, le xylose, le fructose....

Par dérivés des monosaccharides on désigne les produits pouvant être obtenus par déshydratation, isomérisation, réduction ou oxydation :

- des sucres alcools, des alcools et des polyols : en particulier le sorbitol, l'anhydrosorbitol, les hexanetetrols, les hexanetriols, les hexanediols, le xylitol, les pentanetetrols, les pentanetriols, les pentanediols, l'érythritol, les butanetriols, les butanediols, le glycérol, le 1,3-propanediol, le propylène glycol, l'éthylène glycol, les hexanols, les pentanols, les butanols, les propanols, l'éthanol...
- des cétones, des hexane-diones : la 2,5-hexanedione, l'hydroxyacétone...
- des acides carboxyliques et leurs esters, des lactones : l'acide formique, les formiates d'alkyles, l'acide acétique, les acétates d'alkyles, l'acide hexanoïque, les hexanoates d'alkyles, l'acide lévulinique, les lévulinates d'alkyles, l'acide lactique, les lactates d'alkyles, l'acide glutarique, les glutarates d'alkyles, l'acide 3-hydroxypropanoïque, la 3-hydroxybutyrolactone, la γ-butyrolactone, la γ-valérolactone
- des éthers cycliques : par exemple le tétrahydrofurane (THF), le 3-méthyltétrahydrofurane (Me-THF) et ses isomères de position, le 2,4-diméthyltétrahydrofurane et ses isomères de position, le tétrahydropyrane-2-méthanol et ses isomères de position.
- des furanes : l'acide furane-2,5-dicarboxylique, le 5-(hydroxyméthyl)furfural, le furfural...

Par polymères solubles, on désigne tous les produits issus de la condensation entre les monosaccharides, les oligosaccharides et/ou les dérivés des monosaccharides.

A l'issue de la réaction, le milieu réactionnel est prélevé et centrifugé. Le liquide réactionnel est ensuite analysé par chromatographie liquide sous haute pression (HPLC) et par chromatographie gazeuse (GC) en utilisant la réfractométrie pour déterminer la teneur en produits de conversion de la solution aqueuse.

La quantité des produits de réaction solubles dans l'eau (monosaccharides et dérivés, oligosaccharides, polymères solubles) est déterminée par l'analyse COT (Carbone Organique Total) qui consiste en la mesure du carbone en solution. La quantité des monosaccharides et leurs dérivés est déterminée par analyses HPLC.

**EXEMPLES**

Dans les exemples ci-dessous, le catalyseur zircone tungsté ($ZrO_2$-$WO_x$) est commercial et fourni par la société MEL Chemical et contient 10,3% de tungstène en poids.

Le chlorure de fer hexahydraté est commercial et utilisé sans purification.

Exemple 1 : Préparation du catalyseur C1: 0.5%pds Pt/$ZrO_2$-$WO_x$

Une solution aqueuse d'acide hexachloroplatinique $H_2PtCl_6.xH_2O$ à 1,9% en poids (25mL, 0,475g) est ajoutée à température ambiante au support $ZrO_2$-$WO_x$ (24g) préalablement désorbée sous vide (1h, 100°C). Le mélange est agité pendant une heure puis est ensuite évaporé. Le solide obtenu est ensuite mis à sécher à l'étuve à 110°C pendant 24h. Ensuite le solide est calciné sous débit d'azote sec à la température de 150°C pendant 1h, puis de 250°C pendant 1h, puis de 350°C pendant 3h et enfin de 420°C pendant 4h. Il est ensuite réduit sous flux d'hydrogène à 500°C pendant deux heures. Le catalyseur C1 obtenu contient 0,5 % poids de platine.

Exemple 2 : Transformation de la cellulose sans catalyseur

Cet exemple concerne la conversion de la cellulose sans catalyseur pour la production de produits mono- et polyoxygénés.
Dans un autoclave de 100mL, on introduit 50mL d'eau et 1,3g de cellulose SigmaCell®. L'autoclave est chauffé à 190°C et une pression de 5 MPa d'azote est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.
Les résultats obtenus sont référencés dans le Tableau 1

Exemple 3 : Transformation de la cellulose mettant en oeuvre $ZrO_2$-$WO_x$

Cet exemple concerne la conversion de la cellulose à partir de zircone tungstée commerciale pour la production de produits mono- et polyoxygénés.
Dans un autoclave de 100mL, on introduit 50mL d'eau, 1,3g de cellulose SigmaCell® et 0,55g de zircone tungstée. L'autoclave est chauffé à 190°C et une pression de 5 MPa d'azote est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.
Les résultats obtenus sont référencés dans le Tableau 1

Exemple 4 : Transformation de la cellulose mettant en oeuvre le chlorure de fer hexahydraté seul

Cet exemple concerne la conversion de la cellulose à partir de chlorure de fer hexahydraté pour la production de produits mono- et polyoxygénés.
Dans un autoclave de 100mL, on introduit 50mL d'eau, 1,3g de cellulose SigmaCell® et 0,03 g de chlorure de fer hexahydraté. L'autoclave est chauffé à 190°C et une pression de 5 MPa d'azote est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.
Les résultats obtenus sont référencés dans le Tableau 1

Exemple 5 : Transformation de la cellulose mettant en oeuvre le catalyseur C1 (0.5%pds. Pt/$ZrO_2$-$WO_x$)

Cet exemple concerne la conversion de la cellulose à partir du catalyseur C1 décrit dans l'exemple 1 pour la production de produits mono- et polyoxygénés.
Dans un autoclave de 100mL, on introduit 50mL d'eau, 1,3g de cellulose SigmaCell® et 0,55g de catalyseur C1 introduit sous atmosphère d'azote. L'autoclave est chauffé à 190°C et une pression de 5 MPa d'hydrogèneest introduite. Après 24h de réaction le milieu réactionnel est prélevé et centrifugé. Le liquide réactionnel est ensuite analysé par chromatographie liquide sous haute pression (HPLC) et en chromatographie gazeuse (GC) en utilisant la réfractométrie pour déterminer la teneur en produits de conversion de la solution aqueuse.
Les résultats obtenus sont référencés dans le Tableau 1

Exemple 6 : Transformation de la cellulose mettant en oeuvre le catalyseur C1 (0.5% pds. Pt/ZrO$_2$-WO$_x$) en combinaison avec le chlorure de fer hexahydraté FeCl$_3$.6H$_2$O (conforme)

Cet exemple concerne la conversion de la cellulose à partir d'une combinaison du catalyseur C1 décrit dans l'exemple 1 et le chlorure de fer hexahydraté pour la production de produits mono- et polyoxygénés.

Dans un autoclave de 100mL, on introduit 50mL d'eau, 1,3g de cellulose SigmaCell® 0,03 g de chlorure de fer hexahydraté et 0,55g de catalyseur C1 introduit sous atmosphère d'azote. L'autoclave est chauffé à 190°C et une pression de 5 MPa d'hydrogène est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les résultats obtenus sont référencés dans le Tableau 1

La solubilisation est définie comme le pourcentage de solubilisation de la biomasse ou de la cellulose en solution et est calculée suivant l'équation suivante :

$$Solubilisation\ (\%) = 100 * C_{solubilisé}\ /\ C_{initial}$$

dans laquelle $C_{solubilisé}$ représente la quantité de carbone solubilisé analysée par COT (mg) et $C_{initial}$ la quantité de carbone en début de réaction contenue dans la biomasse ou cellulose solide.

Les rendements des produits sont calculés au moyen de l'analyse HPLC. Les rendements indiqués en un dérivé i sont calculés comme suit :

$$Rdmnt(i) = \frac{C\,(i)\ (g\,/\,l)}{Csolubilisé\ \ (g(C)/l)} \times \frac{n \times M\,(C)\ (g\,/\,mol)}{M\,(i)\ (g\,/\,mol)}$$

où C(i) représente la concentration du dérivé i déterminée par HPLC, n représente le nombre d'atomes de carbones du dérivé i, M(C) la masse molaire de l'atome de carbone et M(i) la masse molaire du dérivé.

Tableau 1 : Solubilisation de cellulose et formation d'humines

| exemples | Catalyseur | Solubilisation à 6h | Solubilisation à 12h | Solubilisation à 24h | Formation d'humines |
|---|---|---|---|---|---|
| 2 | Sans catalyseur | 18% | 22% | 25% | + |
| 3 | ZrW | 35% | 42% | 50% | + |
| 4 | FeCl$_3$ | 50% | 52% | 50% | ++ |
| 5 | Pt/ZrW (C1) | 37% | 50% | 60% | Pas d'humines |
| 6 | FeCl$_3$ + PtZrW (C1) | 80% | 90% | 92% | Pas d'humines |

A titre d'exemple, les rendements carbone en produits mono- et polyoxygénés obtenus par transformation de la cellulose en présence d'une combinaison de FeCl$_3$ et PtZrW sont les suivants : propylène glycol (14%), éthylène glycol (8%), 1,2,6-hexanetriol (8%), 1,2-hexanediol (8%), 2,4-diméthyltétrahydrofurane (8%), 1,2-butanediol (5%), érythritol (3%), glycérol (2%), 3-méthyltétrahydrofurane (3%), éthanol (2%), propanol (2%), 2-méthylpentanol (2%), glucose (1 %), acide lactique (1 %), 1,2-pentanediol (1 %), 1-pentanol (1%), tétrahydropyrane-2-méthanol (1%), méthanol (1%).

Exemple 7 : Transformation de la cellulose mettant en oeuvre le catalyseur C1 (0.5% pds. Pt/ZrO$_2$-WO$_x$) en combinaison avec un catalyseur homogène (conforme)

Cet exemple concerne la conversion de la cellulose à partir d'une combinaison du catalyseur C1 décrit dans l'exemple 1 et d'un sel métallique pour la production de produits mono- et polyoxygénés.

Dans un autoclave de 100mL, on introduit 50mL d'eau, 1,3g de cellulose SigmaCell®, un sel métallique et 0,55g de catalyseur C1 sous atmosphère d'azote. Les sels métalliques sont solubles dans l'eau (T = 25°C, P=P atmosphérique). La masse de sel métallique ajoutée est décrite dans le Tableau 2.

L'autoclave est chauffé à 190°C et une pression de 5 MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.

Les résultats obtenus sont référencés dans le Tableau 2.

Tableau 2 : Solubilisation de cellulose et formation d'humines

| Catalyseur | Masse de sel métallique (g) | Solubilisation à 6h (%) | Solubilisation à 12h (%) | Formation d'humines |
|---|---|---|---|---|
| $FeCl_3$ + PtZrW (C1) | 0.033 | 80 | 90 | Pas d'humines |
| $CrCl_3$ + PtZrW (C1) | 0.034 | 81 | 84 | Pas d'humines |
| $ZnCl_2$ + PtZrW (C1) | 0.007 | 39 | 54 | Pas d'humines |
| $C_UCl_2$ + PtZrW (C1) | 0.011 | 44 | 66 | Pas d'humines |
| $MnCl_2$ + PtZrW (C1) | 0.017 | 34 | 49 | Pas d'humines |
| $AlCl_3$ + PtZrW (C1) | 0.054 | 91 | 96 | Pas d'humines |
| $SnCl_4$ + PtZrW (C1) | 0.026 | 46 | 91 | Pas d'humines |
| $BiCl_3$ + PtZrW (C1) | 0.012 | 26 | 23 | Pas d'humines |
| $LaCl_3$ + PtZrW (C1) | 0.025 | 32 | 91 | Pas d'humines |
| $ZrCl_4$ + PtZrW (C1) | 0.015 | 47 | 69 | Pas d'humines |

Exemple 8 : Transformation d'une biomasse lignocellulosique à base de miscanthus mettant en oeuvre la zircone tunastée seule $ZrO_2$-$WO_x$

Cet exemple concerne la conversion d'une biomasse lignocellulosique à base de miscanthus à partir de zircone tungstée commerciale pour la production de produits mono- et polyoxygénés.

Dans un autoclave de 100mL, on introduit 50mL d'eau, 5,9g de biomasse lignocellulosique à base de miscanthus (équivalent à 1,3g de cellulose) et 0,55g de zircone tungstée. L'autoclave est chauffé à 190°C et une pression de 5 MPa d'azote est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les résultats obtenus sont référencés dans le Tableau 3

Exemple 9 : Transformation d'une biomasse lignocellulosique à base de miscanthus mettant en oeuvre le chlorure de fer hexahydraté seul

Cet exemple concerne la conversion d'une biomasse lignocellulosique à base de miscanthus à partir de chlorure de fer hexahydraté pour la production de produits mono- et polyoxygénés.

Dans un autoclave de 100mL, on introduit 50mL d'eau, 5,9g de biomasse lignocellulosique à base de miscanthus (équivalent à 1,3g de cellulose) et 0,03 g de chlorure de fer hexahydraté. L'autoclave est chauffé à 190°C et et une pression de 5 MPa d'azote est introduite. Après 24h de réaction le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les résultats obtenus sont référencés dans le Tableau 3

Exemple 10 : Transformation d'une biomasse lignocellulosique à base de miscanthus mettant en oeuvre le catalyseur C1 en combinaison avec le chlorure de fer hexahydraté (conforme)

Cet exemple concerne la conversion de la cellulose à partir d'une combinaison du catalyseur C1 décrit dans l'exemple 1 et le chlorure de fer hexahydraté pour la production de produits mono- et polyoxygénés.

Dans un autoclave de 100mL, on introduit 50mL d'eau, 5,9g de biomasse lignocellulosique à base de miscanthus (équivalent à 1,3g de cellulose), 0,03 g de chlorure de fer hexahydraté et 0,55g de catalyseur C1 introduit sous atmosphère d'azote. L'autoclave est chauffé à 190°C et et une pression de 5 MPa d'hydrogène est introduite. Après 24h de réaction le milieu réactionnel est prélevé et centrifugé. Le liquide réactionnel est ensuite analysé par chromatographie liquide sous haute pression (HPLC) et en chromatographie gazeuse (GC) en utilisant la réfractométrie pour déterminer la teneur en produits de conversion de la solution aqueuse.

Les résultats obtenus sont référencés dans le Tableau 3

Tableau 3 : Solubilisation de la biomasse lignocellulosique à base de miscanthus

| exemples | Catalyseur | Solubilisation à 6h et 12h | Solubilisation à 24h | Formation d'humines |
|----------|------------|----------------------------|----------------------|---------------------|
| 8 | ZrW | 28% et 30% | 35% | + |
| 9 | $FeCl_3$ | 66% et 65% | 58% | ++ |
| 10 | $FeCl_3$ + PtZrW (C1) | 95% et 96% | 98% | Pas d'humine |

A titre d'exemple, les rendements carbone en produits mono- et polyoxygénés obtenus par transformation de biomasse lignocellulosique à base de miscanthus en présence d'une combinaison de $FeCl_3$ et PtZrW sont les suivants : propylène glycol (16%), 1,2-hexanediol (16%), éthylène glycol (9%), 1,2-butanediol (6%), 2,4-diméthyltétrahydrofurane (6%), acide acétique (4%), acide lactique (3%), 1,2,6-hexanetriol (3%), érythritol (3%), glycérol (2%), 3-méthyltétrahydrofurane (2%), éthanol (1%), propanol (1%), 2-méthylpentanol (1%), glucose (1%), 1,2-pentanediol (1%), 1-pentanol (1%), tétrahydropyrane-2-méthanol (1%), méthanol (1%).

L'association d'un catalyseur homogène (chlorure de fer hexahydraté) et d'un catalyseur hétérogène (PtZrW) se révèle plus efficace en comparaison avec le catalyseur homogène pris seul et le catalyseur hétérogène pris seul.

- On observe une conversion maximale améliorée de 53% à partir de la combinaison du catalyseur C1 décrit dans l'exemple 1 et du chlorure de fer hexahydraté par rapport au catalyseur C1 décrit dans l'exemple 1 pris seul et une conversion améliorée de 84% par rapport au chlorure de fer hexahydraté pris seul.
- On observe une cinétique de solubilisation accélérée à partir de la combinaison du catalyseur C1 décrit dans l'exemple 1 et du chlorure de fer hexahydraté par rapport au catalyseur C1 décrit dans l'exemple 1 pris seul et par rapport au chlorure de fer hexahydraté pris seul.
- On observe une disparition totale de la formation d'humines à partir de la combinaison du catalyseur C1 décrit dans l'exemple 1 et du chlorure de fer hexahydraté par rapport au chlorure de fer hexahydraté pris seul.

Exemple 11 : Préparation du catalyseur C2 : 2.1 %pds $Pt/ZrO_2$-WOx

Une solution aqueuse d'acide hexachloroplatinique $H_2PtCl_6$ (22mL, 1,05g de Pt) est ajoutée à température ambiante au support $ZrO_2$-$WO_x$ (50g) préalablement désorbée sous vide (1h, 100°C). Le mélange est agité pendant une heure puis le solide obtenu est séché à l'étuve à 120°C pendant 24h. Ensuite lesolide est calciné à la température de 150°C pendant 1h, puis de 250°C pendant 1h, puisde 350°C pendant 3h et enfin de 420°C pendant 4h. Il est ensuite réduit sous flux d'hydrogène à 500°C pendant deux heures. Le catalyseur C2 obtenu contient 2,1 % poids de platine.

Exemple 12 : Préparation de catalyseurs hétérogènes contenant du platine

Une série de catalyseurs sont préparés par imprégnation de supports oxydes simples ou mixtes ou de supports carbonés d'une solution d'acide hexachloroplatinique. La teneur en métal Pt visée est de 0,5% par rapport au poids du catalyseur.

Après imprégnation les catalyseurs ainsi préparés sont séchés 24h à 120°C puis calcinés sous débit d'air à la température de 150°C pendant 1h, puis de 250°C pendant 1h, puis de 350°C pendant 3h et enfin de 420°C pendant 4h. Les catalyseurs sont ensuite réduits sous flux d'hydrogène à 500°Cpendant deux heures.

Le catalyseurs préparés sont décrits dans le Tableau 4.

Tableau 4 : Catalyseurs hétérogènes contenant du platine

| Catalyseur | Support | Teneur en Pt (%. Pds.) |
|------------|---------|------------------------|
| C2 | $ZrO_2$-$WO_x$ | 2.1 |
| C3 | $ZrO_2$ | 0.5 |
| C4 | $SiO_2$-$Al_2O_3$ | 0.5 |
| C5 | $CeO_2$-$ZrO_2$ | 0.5 |
| C6 | $TiO_2$-$WO_x$ | 0.5 |

(suite)

| Catalyseur | Support | Teneur en Pt (%. Pds.) |
|---|---|---|
| C7 | C | 0.5 |
| C8 | $ZnAl_2O_4$ | 0.5 |
| C9 | $ZrO_2$-MoOx | 0.5 |

Exemple 13 : Transformation de la cellulose mettant en oeuvre les catalyseurs C3, C4, C5, C6, C7, C8, C9 en combinaison avec le chlorure de fer hexahydraté (conforme)

Cet exemple concerne la conversion de la cellulose à partir d'une combinaison d'un catalyseur C3, C4, C5, C6, C7, C8, C9 décrits dans l'exemple 8 et d'un sel métallique pour la production de produits mono- et polyoxygénés. Dans un autoclave de 100mL, on introduit 50mL d'eau, 1,3g de cellulose SigmaCell®, 0,03g de chlorure de fer et 0,55g de catalyseur sous atmosphère d'azote. Le chlorure de fer hexahydraté est soluble dans l'eau (T = 25°C P= P atmosphérique).
L'autoclave est chauffé à 190°C et et une pression de5 MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.
Les résultats obtenus sont référencés dans le Tableau 5

Tableau 5 : Solubilisation de cellulose et formation d'humines

| Catalyseurs | Nom du catalyseur | Solubilisation à 6h (%) | Solubilisation à 12h (%) | Formation d'humines |
|---|---|---|---|---|
| $FeCl_3$ +Pt/$ZrO_2$-WOx | $FeCl_3$ +C2 | 72 | 85 | Pas d'humine |
| $FeCl_3$ +Pt/$ZrO_2$ | $FeCl_3$ +C3 | 73 | 84 | Pas d'humine |
| $FeCl_3$ +Pt/$SiO_2$-$Al_2O_3$ | $FeCl_3$ +C4 | 69 | 84 | Pas d'humine |
| $FeCl_3$ +Pt/$CeO_2$-$ZrO_2$ | $FeCl_3$ +C5 | 58 | 79 | Pas d'humine |
| $FeCl_3$ +Pt/$TiO_2$-WOx | $FeCl_3$ +C6 | 51 | 53 | Pas d'humine |
| $FeCl_3$ +Pt/C | $FeCl_3$ +C7 | 65 | 78 | Pas d'humine |
| $FeCl_3$ +Pt/$ZnAl_2O_4$ | $FeCl_3$ +C8 | 50 | 54 | Pas d'humine |
| $FeCl_3$ +Pt/$ZrO_2$-MoOx | $FeCl_3$ +C9 | 65 | 72 | Pas d'humine |

Exemple 14 : Préparation de catalyseurs hétérogène contenant de l'oxyde de zirconium

Une série de catalyseurs est préparée par imprégnation d'un support oxyde de zirconium ($ZrO_2$, monoclinique) avec une solution contenant un métal noble.
Le catalyseur C10 est préparé par imprégnation d'un support oxyde de zirconium (MEL Chemical), d'une solution de $H_2PtCl_6$. Après imprégnation le solide est séché 24h à 120°C puis calciné sous débit d'air à la température de 500°C pendant 4h. Le solide est réduit sous flux d'hydrogène à 500°C pendant deux heures. Le catalyseur C10 contient 1.2 %pds. de platine.
Le catalyseur C11 est préparé par imprégnation d'un support oxyde de zirconium (MEL Chemical) d'une solution de $H_2PdCl_4$. Après imprégnation le solide est séché 24h à 120°C puis calciné sous débit d'air à la température de 450°C pendant 4h. Le solide est réduit sous flux d'hydrogène à 250°C pendant deux heures. Le catalyseur C11 contient 1.1%pds. de palladium.
Le catalyseur C12 est préparé par imprégnation d'un support oxyde de zirconium (MEL Chemical) d'une solution de $H_2IrCl_6$. Après imprégnation le solide est séché 24h à 120°C puis calciné sous débit d'air à la température de 500°C pendant 4h. Le solide est réduit sous flux d'hydrogène à 500°C pendant deux heures. Le catalyseur C12 contient 1.1%pds. d'iridium.

<u>Exemple 15 : Transformation de la cellulose mettant en oeuvre les catalyseurs C10, C11, C12 en combinaison avec le chlorure de fer hexahydraté (conforme)</u>

Cet exemple concerne la conversion de la cellulose à partir d'une combinaison d'un catalyseur C10, C11, C12 décrits dans l'exemple 10 et d'un sel métallique pour la production de produits mono- et polyoxygénés.
Dans un autoclave de 100mL, on introduit 50mL d'eau, 1,5g de cellulose SigmaCell®, 0.03g de chlorure de fer et du catalyseur C10, C11 ou C12 sous atmosphère d'azote. La masse de catalyseur est calculée pour que la quantité de métal noble introduite soit équivalente. Le sel métallique de chlorure de fer hexahydraté est soluble dans l'eau (T = 25°C, P= P atmosphérique).
L'autoclave est chauffé à 190°C et et une pression de 5 MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.

Les résultats obtenus sont référencés dans le Tableau 6.

Tableau 6 : Solubilisation de cellulose et formation d'humines

| Catalyseurs | Masse de catalyseur | Solubilisation à 6h (%) | Solubilisation à 12h (%) | Formation d'humines |
|---|---|---|---|---|
| Sans catalyseur | 0 | 50 | 52 | oui |
| FeCl$_3$ +Pt/ZrO$_2$ (C10) | 0.55g | 78 | 82 | Pas d'humines |
| FeCl$_3$ +Pd/ZrO$_2$ (C11) | 0.27g | 52 | 62 | Pas d'humines |
| FeCl$_3$ +Ir/ZrO$_2$ (C12) | 0.55g | 61 | 73 | Pas d'humines |

<u>Exemple 16 : Préparation de catalyseurs hétérogène contenant de l'oxyde de silicium et d'aluminium.</u>

Une série de catalyseurs est préparé par imprégnation d'un support silice-alumine.
Le catalyseur C13 est préparé par imprégnation d'un support silice alumine (Siralox 30, Sasol) d'une solution d'acide hexachloroplatinique. Après imprégnation le solide est séché 24h à 120°C puis calciné sous débit d'air à à température de 500°C pendant 4h.
Le solide est réduit sous flux d'hydrogène à 500°Cpendant deux heures. Le catalyseur C13 contient 0.7 %pds. de platine.
Le catalyseur C14 est préparé par imprégnation d'un support silice alumine (Siralox 30, Sasol) d'une solution de d'acétylacétone de ruthénium Ru(acac)$_3$ dissout dans de l'acétone. Après imprégnation le solide est séché 24h à 120°C puis calciné sous débit d'air à la température de 300°C pendant 4h. Le solde est réduit sous flux d'hydrogène à 300°C pendant deux heures. Le catalyseur C14 content 1.2 %pds. de ruthénium.
Le catalyseur C15 est préparé par imprégnation du catalyseur C13 d'une solution de chlorure d'étain SnCl2. Après imprégnation le solide est séché 24h à 120°C puis calciné sous débit d'air à la température de 550°C pendant 4h. Le catalyseur C15 contient 0.7 %pds. de platine et 1.5%pds. d'étain.
Le catalyseur C16 est préparé par imprégnation d'un support silice alumine (Siralox 30, Sasol) d'une solution de nitrate de nickel. Après imprégnation le solide est séché 24h à 120°C puis calciné sous débit d'air à la température de 500°C pendant 4h. Le solide est réduit sous flux d'hydrogène à 500°C pendant deux heures. Le catalyseur C16 contient 3.6%pds. de nickel.
Le catalyseur C17 est préparé par imprégnation du catalyseur C16 d'une solution chlorure d'étain SnCl$_2$. Après imprégnation le solide est séché 24h à 120°C puis calciné sous débit d'air à la température de 550°C pendant 4h. Le solide est réduit sous flux d'hydrogène à 500°C pendant deux heures. Le catalyseur C17 contient 3.6%pds. de nickel et 1.3%pds. d'étain.

<u>Exemple 17 : Transformation de la cellulose mettant en oeuvre les catalyseurs C13, C14, C15, C16 et C17 en combinaison avec le chlorure de fer hexahydraté (conforme)</u>

Cet exemple concerne la conversion de la cellulose à partir d'une combinaison d'un ou plusieurs catalyseurs hétérogènes choisis parmi C13, C14, C15, C16 et C17 décrits dans l'exemple 12 et d'un sel métallique de chlorure de fer pour la production de produits mono- et polyoxygénés.
Dans un autoclave de 100mL, on introduit 50mL d'eau, 1,5g de cellulose SigmaCell®, 0.03g de chlorure de fer et du catalyseur C13, C14, C15, C16 ou C17 sous atmosphère d'azote. Le sel métallique de chlorure de fer hexahydraté est soluble dans l'eau (T = 25°C, P= P atmosphérique).

L'autoclave est chauffé à 185°C et une pression de 5 MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.

Les résultats obtenus sont référencés dans le Tableau 7.

Tableau 7 : Solubilisation de cellulose et formation d'humines

| Catalyseur Hétérogène | $m_{catalyseur}$ (g) | Solubilisation à 6h (%) | Solubilisation à 12h (%) | Formation d'humines |
|---|---|---|---|---|
| $FeCl_3$ +Pt/$SiO_2$-$Al_2O_3$(C13) | 0.5 | 78 | 98 | Pas d'humines |
| $FeCl_3$ +Ru/$SiO_2$-$Al_2O_3$ (C14) | 0.30 | 62 | 82 | Pas d'humines |
| $FeCl_3$ +Ni/$SiO_2$-$Al_2O_3$ (C15) | 1.0 | 30 | 45 | Pas d'humines |
| $FeCl_3$ +PtSn/$SiO_2$-$Al_2O_3$ (C16) | 1.9 | 70 | 90 | Pas d'humines |
| $FeCl_3$ +NiSn/ $SiO_2$-$Al_2O_3$ (C17) | 0.9 | 50 | 61 | Pas d'humines |
| $FeCl_3$+Ru/$SiO_2$-$Al_2O_3$+Pt/$SiO_2$-$Al_2O_3$(C13 + C14) | 0.30+0.09 | 68 | 86 | Pas d'humines |

Exemple 18 : Transformation de la cellulose mettant en oeuvre le catalyseur C1 en combinaison avec le chlorure de fer hexahydraté (conforme) dans des conditions opératoires variables.

Cet exemple concerne la conversion de la cellulose à partir d'une combinaison d'un catalyseur hétérogène C1 et d'un sel métallique de chlorure de fer pour la production de produits mono- et polyoxygénés.

Dans un autoclave de 100mL, on introduit 50mL d'eau, 1,5g de cellulose SigmaCell®, 0.03g de chlorure de fer et 0.5 g du catalyseur C1 sous atmosphère d'azote. Le sel métallique de chlorure de fer hexahydraté est soluble dans l'eau (T = 25°C, P= P atmosphérique).

L'autoclave est chauffé à une température T comprise entre 150°C et 250°C et une pression P variant entre 5 MPa et 8MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.

Les résultats obtenus sont référencés dans le Tableau 8.

Tableau 8 : Solubilisation de cellulose et formation d'humines

| Température (°C) | Pression (MPa) | Solubilisation à 6h (%) | Solubilisation à 12h (%) | Formation d'humines |
|---|---|---|---|---|
| 150 | 0,50 | 10 | 11 | Pas d'humines |
| 170 | 0,50 | 18 | 22 | Pas d'humines |
| 190 | 0,50 | 72 | 85 | Pas d'humines |
| 190 | 0,80 | 75 | 91 | Pas d'humines |
| 210 | 0,50 | 76 | 95 | Pas d'humines |
| 245 | 0,50 | 72 | 60 | Pas d'humines |

Exemple 19 : Transformation de glucose mettant en oeuvre le catalyseur C1 (0.5% pds. Pt/$ZrO_2$-$WO_x$) en combinaison avec un catalyseur homogène (conforme)

Cet exemple concerne la conversion de glucose à partir d'une combinaison du catalyseur C1 décrit dans l'exemple 1 et d'un sel métallique pour la production de produits mono- et polyoxygénés.

Dans un autoclave de 100mL, on introduit 50mL d'eau, 1,3g de glucose, un sel métallique et 0,55g de catalyseur C1 sous atmosphère d'azote. Les sels métalliques sont solubles dans l'eau (T = 25°C, P= P atmosphérique). La masse de sel métallique ajoutée est décrite dans le Tableau 2.

L'autoclave est chauffé à 190°C et et une pression de 5 MPa d'hydrogène est introduite. Après 12h de réaction le milieu réactionnel est prélevé et centrifugé. Des prélèvements sont également effectués au cours du tests et analysés par chromatographie liquide sous haute pression (HPLC) en utilisant la réfractométrie pour déterminer la teneur en produits de conversion de la solution aqueuse.

Les résultats obtenus sont référencés dans le Tableau 9.

Tableau 9 : Transformation de glucose et formation d'humines

| Sel métallique | Masse de sel métallique (g) | Conversion à 6h (%) | Conversion à 12h (%) | Formation d'humines |
|---|---|---|---|---|
| Pt/ZrW (C1) + FeCl$_3$ | 0,033 | 72 | 100 | Pas d'humines |

## Revendications

1. Procédé de transformation de biomasse lignocellulosique ou de cellulose en composés mono ou polyoxygénés, dans lequel la biomasse lignocellulosique ou la cellulose est mise en contact, simultanément, avec une combinaison d'un ou plusieurs catalyseurs homogènes et d'un ou plusieurs catalyseurs hétérogènes, dans une même enceinte réactionnelle, en présence d'au moins un solvant, ledit solvant étant de l'eau seule ou en mélange avec au moins un autre solvant, sous atmosphère réductrice, et à une température comprise entre 80°C et 250°C, et à une pression comprise entre 0,5 MPa et 20 MPa,

    - ledit ou lesdits catalyseur(s) homogène(s) comprenant un sel métallique hydraté ou non de formule générale MX$_n$.n'H$_2$O dans lequel M est un métal choisi parmi les métaux des groupes 3 à 16 de la classification périodique, n est un nombre entier compris entre 1 et 6 et n' est un nombre entier compris entre 0 et 6 et X est au moins un anion choisi parmi les halogénures, les nitrates, les carboxylates, les halogénocarboxylates, les acétylacétonates, les alcoolates, les phénolates, substitués ou non, les sulfates, les alkylsulfates, les phosphates, les alkylphosphates, les halogénosulfonates, les alkylsulfonates, les perhalogénoalkylsulfonates, les bis(perhalogénoalkylsulfonyl)amidures, les arènesulfonates, substitués ou non par des groupements halogènes ou halogénoalkyles, lesdits anions X pouvant être identiques ou différents dans le cas où n est supérieur à 1,
    - ledit ou lesdits catalyseur(s) hétérogène(s) comprenant au moins un métal choisi parmi les métaux des groupes 6 à 11 et les métaux du groupe 14 de la classification périodique et un support choisi parmi les oxydes des éléments choisis parmi l'aluminium, le titane, le silicium, le zirconium, le cérium, et le niobium, et les oxydes mixtes choisis parmi les aluminates de zinc, de cuivre et de cobalt, lesdits oxydes pouvant être dopés ou non par au moins un composé métallique choisi parmi le tungstène, l'étain, le molybdène et l'antimoine, pris seuls ou en mélange, les aluminosilicates cristallisés ou non, les aluminophosphates et les composés carbonés amorphes ou cristallisés.

2. Procédé selon la revendication 1 dans lequel le métal M du ou des catalyseurs homogènes est choisi parmi les métaux suivants : Cr, Mn, Fe, Co, Ni, Cu, Zn, Al, Ga, In, Cu, Zr, Bi, La et Sn.

3. Procédé selon l'une des revendications 1 ou 2 dans lequel le métal M ou des catalyseurs homogènes est choisi parmi les métaux suivants : Cr, Mn, Fe, Zn, Al, Sn, Cu, Zr, Bi et La.

4. Procédé selon l'une des revendications 1 à 3 dans lequel l'anion X du ou des catalyseurs homogènes est au moins un anion choisi parmi les halogénures, les alkylsulfonates, les perhalogénoalkylsulfonate, les bis(perhalogénoalkylsulfonyl)amidures.

5. Procédé selon la revendication 4 dans lequel l'anion X du ou des catalyseurs homogènes est un chlorure dans le cas où n est égal à 3.

6. Procédé selon l'une des revendications 1 à 5 dans lequel le métal du ou des catalyseurs hétérogènes est choisi parmi les métaux Mo, W, Re, Ru, Co, Rh, Ir, Ni, Pd, Pt, Cu, d'une part et Sn, d'autre part, pris seuls et en mélange

7. Procédé selon la revendication 6 dans lequel le métal du ou des catalyseurs hétérogènes est le platine.

8. Procédé selon l'une des revendications 1 à 7 dans lequel la teneur en métal sur ledit ou lesdits catalyseurs hétérogènes est comprise entre 0,01 et 10% poids par rapport à la masse totale du ou desdits catalyseurs.

9. Procédé selon l'une des revendications 1 à 8 dans lequel le ou les catalyseur(s) homogène(s) sont introduits dans l'enceinte réactionnelle à raison d'une quantité correspondant à un rapport massique biomasse/catalyseur(s) homogène(s) compris entre 1 et 1000.

10. Procédé selon l'une des revendications 1 à 8 dans lequel, dans le cas où ledit procédé opère en présence d'eau en mélange avec au moins un autre solvant, le mélange de solvants comprend une teneur massique en eau supérieure à 5% poids et de manière préférée supérieure à 30% et de manière très préférée supérieure à 50% par rapport à la masse totale dudit mélange.

11. Procédé selon l'une des revendications 1 à 10 dans lequel ledit procédé opère uniquement en présence d'eau.

12. Procédé selon l'une des revendications 1 à 11 dans lequel le procédé opère en présence d'au moins un solvant à l'exception des solvants choisis parmi les liquides ioniques.

13. Procédé selon l'une des revendications précédentes l'atmosphère réductrice est une atmosphère d'hydrogène, pure ou en mélange.

14. Procédé selon l'une des revendications précédentes opérant à une température comprise entre 150°C et 240°C, et à une pression comprise entre 2 MPa et 10 MPa.

## Patentansprüche

1. Verfahren zur Umwandlung von Lignocellulose-Biomasse oder Cellulose aus mono- oder polyoxygenierten Verbindungen, wobei die Lignocellulose-Biomasse oder die Cellulose gleichzeitig mit einer Kombination eines oder mehrerer homogener Katalysatoren und eines oder mehrerer heterogener Katalysatoren in demselben Reaktionsbehälter in Gegenwart mindestens eines Lösungsmittels, wobei das Lösungsmittel nur Wasser oder in Mischung mit mindestens einem anderen Lösungsmittel ist, unter einer Reduktionsatmosphäre und bei einer Temperatur zwischen 80 °C und 250 °C und bei einem Druck zwischen 0,5 MPa und 20 MPa in Kontakt gebracht wird,

 - wobei der oder die homogene(n) Katalysator(en) ein hydratisiertes oder nicht hydratisiertes Metallsalz der allgemeinen Formel $MX_n.n'H_2O$ ist (sind), wobei M ein Metall, ausgewählt aus Metallen der Gruppen 3 bis 16 des Periodensystems ist, n eine ganze Zahl zwischen 1 und 6 ist, und n' eine ganze Zahl zwischen 0 und 6 ist, und X mindestens ein Anion ist, ausgewählt aus Halogeniden, Nitraten, Carboxylaten, Halogencarboxylaten, Acetylacetonaten, Alkoholaten, Phenolaten, substituiert oder nicht, Sulfaten, Alkylsulfaten, Phosphaten, Alkylphosphaten, Halogensulfonaten, Alkylsulfonaten, Perhalogenalkylsulfonaten, Bis-(perhalogenalkylsulfonyl)-amiden, Arensulfonaten, substituiert mit Halogen- oder Halogenalkyl-Gruppen oder nicht, wobei die Anionen X identisch oder verschieden sein können in dem Fall, wo n größer als 1 ist,
 - wobei der oder die heterogene(n) Katalysator(en) umfasst (umfassen): mindestens ein Metall, ausgewählt aus den Metallen der Gruppen 6 bis 11 und den Metallen der Gruppe 14 des Periodensystems, und einen Träger, ausgewählt aus Oxiden von Elementen, ausgewählt aus Aluminium, Titan, Silicium, Zirconium, Cer und Niob, und gemischten Oxiden, ausgewählt aus Zink-, Kupfer- und Kobaltaluminaten, wobei die Oxide dotiert sein können oder nicht mit mindestens einer Metallverbindung, ausgewählt aus Wolfram, Zinn, Molybdän und Antimon, allein oder in Mischung, kristallisierten oder nicht kristallisierten Aluminosilikaten, Aluminophosphaten und amorphen oder kristallisierten kohlenstoffhaltigen Verbindungen.

2. Verfahren nach Anspruch 1, wobei das Metall M des homogenen Katalysators oder der homogenen Katalysatoren aus den folgenden Metallen ausgewählt wird: Cr, Mn, Fe, Co, Ni, Cu, Zn, Al, Ga, In, Cu, Zr, Bi, La und Sn.

3. Verfahren nach einem der Ansprüche 1 oder 2, wobei das Metall des homogenen Katalysators oder der homogenen Katalysatoren aus den folgenden Metallen ausgewählt wird: Cr, Mn, Fe, Zn, Al, Sn, Cu, Zr, Bi und La.

4. Verfahren nach einem der Ansprüche 1 bis 3, wobei das Anion X des homogenen Katalysators oder der homogenen Katalysatoren mindestens ein Anion ist, ausgewählt aus Halogeniden, Alkylsulfonaten, Perhalogenalkylsulfonat, Bis-(perhalogenalkylsulfonyl)-amiden.

5. Verfahren nach Anspruch 4, wobei das Anion X des homogenen Katalysators oder der homogenen Katalysatoren ein Chlorid ist in dem Fall, wo n gleich 3 ist.

6. Verfahren nach einem der Ansprüche 1 bis 5, wobei das Metall des heterogenen Katalysators oder der heterogenen Katalysatoren ausgewählt wird aus den Metallen Mo, W, Re, Ru, Co, Rh, Ir, Ni, Pd, Pt, Cu einerseits und Sn andererseits, allein oder in Mischung.

**7.** Verfahren nach Anspruch 6, wobei das Metall des heterogenen Katalysators oder der heterogenen Katalysatoren Platin ist.

**8.** Verfahren nach einem der Ansprüche 1 bis 7, wobei der Metallgehalt des heterogenen Katalysators oder der heterogenen Katalysatoren zwischen 0,01 und 10 Gew.-%, bezogen auf die Gesamtmasse des Katalysators oder der Katalysatoren, beträgt.

**9.** Verfahren nach einem der Ansprüche 1 bis 8, wobei der oder die homogene(n) Katalysator(en) in den Reaktionsbehälter in einer Menge eingebracht wird oder werden, die einem Massenverhältnis Biomasse/homogener (homogene) Katalysator(en) zwischen 1 und 1000 entspricht.

**10.** Verfahren nach einem der Ansprüche 1 bis 8, wobei in dem Fall, wo das Verfahren in Gegenwart einer Mischung mit mindestens einem anderen Lösungsmittel betrieben wird, die Mischung von Lösungsmitteln einen Massengehalt an Wasser von mehr als 5 Ges.-% und bevorzugt mehr als 30 Ges.-% und besonders bevorzugt mehr als 50 Ges.-%, bezogen auf die Gesamtmasse der Mischung, umfasst.

**11.** Verfahren nach einem der Ansprüche 1 bis 10, wobei das Verfahren nur in Gegenwart von Wasser betrieben wird.

**12.** Verfahren nach einem der Ansprüche 1 bis 11, wobei das Verfahren in Gegenwart mindestens eines Lösungsmittels betrieben wird, mit Ausnahme von Lösungsmitteln, die aus ionischen Flüssigkeiten ausgewählt werden.

**13.** Verfahren nach einem der vorhergehenden Ansprüche, wobei die Reduktionsatmosphäre eine Wasserstoffatmosphäre, rein oder in Mischung, ist.

**14.** Verfahren nach einem der vorhergehenden Ansprüche, welches bei einer Temperatur zwischen 150 °C und 240 °C und bei einem Druck zwischen 2 MPa und 10 MPa betrieben wird.

## Claims

**1.** A process for the transformation of lignocellulosic biomass or cellulose into mono- or poly-oxygenated compounds, in which the lignocellulosic biomass or the cellulose is brought into simultaneous contact with a combination of one or more homogeneous catalysts and one or more heterogeneous catalysts in the same reaction chamber, in the presence of at least one solvent, said solvent being water alone or as a mixture with at least one other solvent, in a reducing atmosphere, and at a temperature in the range 80°C to 250°C and at a pressure in the range 0.5 MPa to 20 MPa,

- said homogeneous catalyst or catalysts comprising a metallic salt which may or may not be hydrated with general formula $MX_n.n'H_2O$ in which M is a metal selected from the metals from groups 3 to 16 of the periodic classification, n is a whole number in the range 1 to 6 and n' is a whole number in the range 0 to 6 and X is at least one anion selected from halides, nitrates, carboxylates, halocarboxylates, acetylacetonates, alcoholates, phenolates, which may or may not be substituted, sulphates, alkylsulphates, phosphates, alkylphosphates, halosulphonates, alkylsulphonates, perhaloalkylsulphonates, bis(perhaloalkylsulphonyl)amides, arenesulphonates, which may or may not be substituted with halogen or haloalkyl groups, said anions X possibly being identical or different in the case in which n is greater than 1,
- said heterogeneous catalyst or catalysts comprising at least one metal selected from metals from groups 6 to 11 and metals from group 14 of the periodic classification and a support selected from oxides of elements selected from aluminium, titanium, silicon, zirconium, cerium and niobium, and mixed oxides selected from zinc, copper and cobalt aluminates, said oxides possibly being doped or not doped with at least one metallic compound selected from tungsten, tin, molybdenum and antimony, used alone or as a mixture, crystalline or non-crystalline aluminosilicates, aluminophosphates and amorphous or crystalline carbon compounds.

**2.** The process according to claim 1, in which the metal M of the homogeneous catalyst or catalysts is selected from the following metals: Cr, Mn, Fe, Co, Ni, Cu, Zn, Al, Ga, In, Cu, Zr, Bi, La and Sn.

3. The process according to one of claims 1 or 2, in which the metal M of the homogeneous catalyst or catalysts is selected from the following metals: Cr, Mn, Fe, Zn, Al, Sn, Cu, Zr, Bi and La.

4. The process according to one of claims 1 to 3, in which the anion X of the homogeneous catalysts is at least one anion selected from halides, alkylsulphonates, perhaloalkylsulphonates, and bis(perhaloalkylsulphonyl)amides.

5. The process according to claim 4, in which the anion X of the homogeneous catalyst or catalysts is a chloride in the case in which n is equal to 3.

6. The process according to one of claims 1 to 5, in which the metal of the heterogeneous catalyst or catalysts is selected from the metals Mo, W, Re, Ru, Co, Rh, Ir, Ni, Pd, Pt, Cu, on the one hand and Sn, on the other hand, alone or as a mixture.

7. The process according to claim 6, in which the metal of the heterogeneous catalyst or catalysts is platinum.

8. The process according to one of claims 1 to 7, in which the quantity of metal on said heterogeneous catalyst or catalysts is in the range 0.01% to 10% by weight with respect to the total mass of said catalyst or catalysts.

9. The process according to one of claims 1 to 8, in which the homogeneous catalyst or catalysts are introduced into the reaction chamber in a quantity corresponding to a biomass/homogeneous catalyst(s) ratio in the range 1 to 1000 by weight.

10. The process according to one of claims 1 to 8 in which, in the case in which said process is operated in the presence of water mixed with at least one other solvent, the mixture of solvents comprises a quantity by weight of water of more than 5% by weight and preferably more than 30% by weight, more preferably more than 50% with respect to the total mass of said mixture.

11. The process according to one of claims 1 to 10, in which said process is operated in the presence of water alone.

12. The process according to one of claims 1 to 11, in which the process operates in the presence of at least one solvent with the exception of solvents selected from ionic liquids.

13. The process according to one of the preceding claims, in which the reducing atmosphere is an atmosphere of hydrogen, pure or as a mixture.

14. The process according to one of the preceding claims, operated at a temperature in the range 150°C to 240°C, and at a pressure in the range 2 MPa to 10 MPa.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20090326286 A
- WO 2012035160 A
- US 2011313208 A

**Littérature non-brevet citée dans la description**

- **SELS et al.** *Chem. Comm.,* 2010, vol. 46, 3577-3579
- **ZHANG et al.** *Chem. Comm.,* 2012, vol. 48, 7052-7054
- **C. LIANG.** *Green Chem.,* 2013, vol. 15, 891-895
- **R. RAINES.** *JACS,* 2009, vol. 131, 1979-1985
- **MAKKEE et al.** *Catal. Sci. Technol.*
- Handbook of Chemistry and Physics. 1995